# EUROPEAN PATENT APPLICATION

(11) **EP 1 709 901 A2**
(43) Date of publication of application: **11.10.2006**
(21) Application number: 06111904.6
(22) Date of filing: 29.03.2006
(51) Int. Cl.: A61B 1/05, A61B 5/00, A61B 5/07

(54) **System and method for performing capsule endoscopy in remote sites**

(30) Priority: 06.04.2005 US 668602 P
(71) Applicant: Given Imaging Ltd., Yoqneam 20692 (IL)
(72) Inventor: Frisch, Mordechai, 20186, Moreshet (IL); Rippel, Amir, 34987, Haifa (IL); Gerber, Jeremy Pinchas, 42755, Netanya (IL); Klevens, Alan I., Roswell GA, GA Georgia 30076 (US)
(74) Representative: Dr. Graf & Partner

(57) **Abstract**

A central site may provide downloading and processing services for a plurality of satellite sites that may perform capsule endoscope procedures. Processing may include reviewing image data acquired by a capsule endoscope and performing an at least partial analysis or review of the data reviewed. Health professions and/or physician in the satellite sites may prepare a report or diagnosis on the patient's condition based on the review and analysis performed by the central site

## Description

### FIELD OF THE INVENTION

The present invention relates to in-vivo sensing for the diagnosis of a condition of the GI tract. More specifically the present invention relates to a system and method to at least partially perform diagnosis of a GI condition in a remote location.

### BACKGROUND OF THE INVENTION

Recently, swallowable sensing devices, for example, imaging devices have been used for diagnosis of gastrointestinal (GI) conditions. The swallowable sensing device may typically provide imaging capability and a wireless data transfer capability. Typically, the sensing device may be configured to sequentially capture images of GI tract, (e.g. esophagus, stomach, small intestine, colon) while passively advancing through the GI tract and finally being naturally excreted from the human body.

Image data taken in a body by the swallowable sensing device may be sequentially transmitted outside through, for example, radio communication and may be stored in a memory. A patient may for example carry a receiver that may have a radio communication capability and a memory capability. The patient may freely perform normal actions during the observation period that may begin after swallowing of the sensing device and end upon its excretion. After observation, a user, for example, a health professional may download images captured in-vivo and perform analysis of the GI condition for diagnosis purposes.

### SUMMARY OF THE INVENTION

In one embodiment of the present invention a system and method may be provided where capturing of in-vivo data may be performed in one or more satellite sites and downloading and processing of data from each of the satellite sites may be performed in a typically single central site.

In some embodiments of the present invention the in-vivo data may be image data. In other embodiments of the present invention, the in-vivo data may be additional and/or other data from the GI tract.

In some embodiments of the present invention, data captured in-vivo may be wirelessly transmitted to an external recording device. In other embodiments of the present invention, the recording device may be equipped with a removable memory unit.

In some embodiments, there is provided a method for performing capsule endoscopy on a patient located at a satellite site remote from a central site, comprising the steps of:
initializing a recording device located external to the patient;
obtaining in-vivo data of the patient from a capsule swallowed by the patient, the in-vivo data being transmitted wirelessly from the capsule to the recording device;
providing the in-vivo data from the recording device to the central site; and
analyzing the in-vivo data at the central site only.

In some embodiments, the step of providing the in-vivo data to the central site comprises transporting the recording device or a removable and/or mobile memory device which contains information from the recording device from the satellite site to the central site.

In other embodiments, the step of providing the in-vivo data to the central site comprises transmitting the in-vivo data from the recording device at the satellite site to the central site via the Internet, or wirelessly.

In some embodiments, the in-vivo data is transferred from the recording device to a portable memory device.

In some embodiments, the step of initializing the recording device comprises entering patient information data on the recording device.

In some embodiments, there may also be provided a system for performing capsule endoscopy, comprising:
a satellite site at which a patient is located, the satellite site being provided with software for performing initialization of recording devices;
a recording device that has undergone an initialization process at the satellite site and
which is located external to the patient, for receiving wirelessly and for storing in-vivo data generated by a capsule swallowed by the patient; and
a central site remote from the satellite site for analyzing in-vivo data stored in the recording device, only the central site being provided with software for analyzing the in-vivo data received from the satellite site.

In some embodiments, the recording device is transferred to the central site after the storing of in-vivo data generated by the capsule swallowed by the patient at the satellite site.
In some embodiments, the system further comprises a portable memory device for receiving in-vivo data from the recording device.

### BRIEF DESCRIPTION OF THE DRAWINGS

The subject matter regarded as the invention is particularly pointed out and distinctly claimed in the detailed description in the specification. The invention, however, may best be understood by reference to the following detailed description when read with the accompanied drawings in which:
Figure 1A is a block diagram of an in vivo data sensing system provided in a satellite site according to some embodiments of the present invention;
Figure 1B is a block diagram of an in-vivo data downloading and processing system provided in a central site according to some embodiments of the present invention;
Figure 2A is a schematic illustration of a system provided in a satellite site to capture and record in-vivo image data obtained from a swallowable sensing device according to an embodiment of the present invention;
Figure 2B is a schematic illustration of a system provided in a central site for downloading and processing data captured in-vivo according to one embodiment of the present invention;
Figure 3 is flow chart describing a method for recording in-vivo data in a satellite site and downloading and processing the in-vivo data in a central site according to an embodiment of the present invention; and
Figure 4 is a block diagram of a method and system of diagnosis of a GI condition where data acquisition is performed in a plurality of satellite sites and downloading and processing of the acquired data may be performed in a central site.

It will be appreciated that for simplicity and clarity of illustration, elements shown in the figures have not necessarily been drawn accurately or to scale. For example, the dimensions of some of the elements may be exaggerated relative to other elements for clarity, or several physical components may be included in one functional block or element. Further, where considered appropriate, reference numerals may be repeated among the figures to indicate corresponding or analogous elements.

### DETAILED DESCRIPTION OF THE INVENTION

In the following description, various aspects of the present invention will be described. For purposes of explanation, specific configurations and details are set forth in order to provide a thorough understanding of the present invention. However, it will also be apparent to one skilled in the art that the present invention may be practiced without the specific details presented herein. Furthermore, well known features may be omitted or simplified in order not to obscure the present invention.

Performing in-vivo diagnosis of the GI tract, with for example, a swallowable imaging device, e.g. capsule endoscope may be more cost effective and accessible in remote rural areas with long travel distances and in places where population may be scattered if the data acquisition of the procedure may be done at a satellite site in proximity to the patient while either all or some of the downloading, processing, reading and/or interpretation of the procedure data may be performed in an alternate central review site. Data acquired from known swallowable imaging devices may include for example thousands of frames of image data acquired over long periods of time, e.g. six to eight hours. Other suitable periods of data acquisition and number of frames may be used. Downloading and reviewing the acquired data may be time consuming and may require purchasing of dedicated and expensive equipment and software. In addition, successful diagnosis based on the acquired data may require special training of the health professional performing the procedure. In some embodiments of the present invention, the downloading and reviewing procedure may that may have trained health professionals experienced in analyzing the data acquired, i.e. in-vivo image data of the GI tract.

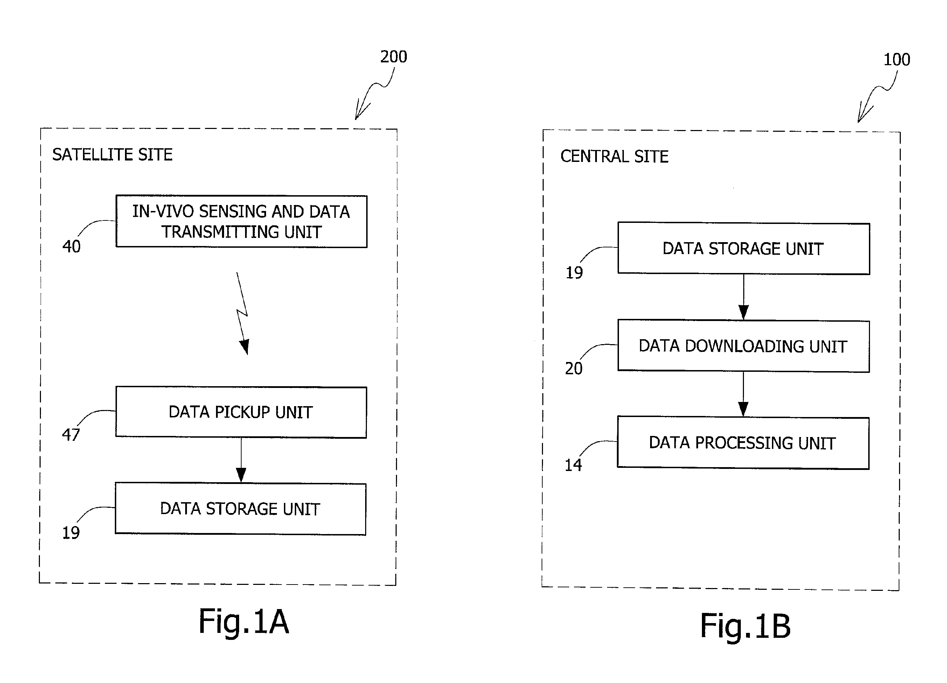

Reference is now made to Fig. 1A showing a block diagram of an in vivo data capture system provided in a satellite site according to an embodiment of the present invention. According to some embodiments of the present invention, satellite system 200 may include, for example, an in-vivo sensing unit and/or device 40 that may transmit captured sensed data by, for example wireless connection. In some embodiments of the present invention and typically, a data pickup unit 47 may pick up the in-vivo data transmitted and store it in data storage unit 19. Typically, in- vivo sensing unit 40 may be, for example, an in-vivo imaging device that may capture frames of image data while passing through, for example, the GI tract. Other in-vivo sensing units that sense other data besides or in addition to image data may be used and incorporated in the satellite system 200. For example, pH data, pressure data, temperature data, biological and/or physiological data and other data from within a body lumen may be sensed, transmitted and recorded according to embodiments of the invention. In some embodiments of the present invention, data sensed by the in-vivo sensing unit 40 may be wirelessly transmitted. In other embodiments of the present invention some or all the in-vivo sensed data may be stored in the in-vivo device. In yet other embodiments of the present invention, data sensed or captured by the in-vivo sensing unit 40 may be transmitted externally through for example, radio communication. Other methods of wireless communications may be used. Typically, radio waves transmitted from the in-vivo sensing device may be picked up by the data pickup unit 47 that may include for example, one or more antennas 17 that may, for example, be positioned in the vicinity of the patient, e.g. around the patient. According to some embodiments of the present invention, the data pickup unit 47 may also include a signal selector and/or combiner that may select and or combine one or more signals from the data signals that are picked up by the antennas 17. In one example signal selector and/or combiner may be a multiplexer. The data pickup unit 47 may also include an amplifier and the selected signal may undergo amplification by the amplifier. Data acquired may be stored in a storage unit 19, for example a portable storage unit that may be worn by the patient. Other suitable configurations for the data pickup unit 47 and the data storage unit 19 may be implemented.

Reference is now made to Fig. 1B showing a block diagram of an in-vivo data downloading and processing system provided in a central site according to an embodiment of the present invention. Central system 100 may be equipped, for example, with a data downloading unit 20 and a data processing unit 14 that may download and process data captured by in-vivo sensing device 40. Storage unit 19 may be transferred from the satellite system 200 to the central system 100 by for example wireless transmission, mail or hand delivery. According to some embodiments data from storage unit 19 may be copied onto a portable storage device (e.g., a disc-on-key or other erasable or non erasable memories), which may be sent to the central system for further processing and/or analysis. Data from the storage unit 19 may be downloaded and processed in the central site. According to one embodiment of the present invention, processing of data may include, for example, converting stored data to a video stream that may be saved for example, on a CD and returned to the satellite site for review and/or physician analysis. In another embodiment of the present invention and typically, data processing may include some initial analysis of the stored data that may be sent by for example on a disk back to the satellite site, for preparation of a fmal report and diagnosis. In yet other embodiments, the complete analysis of the data may be performed in the central site and a report may be sent to the satellite site. Other suitable methods of processing may be performed by the central site system 100. Data other than image data may be processed in the central site. For example, according to one embodiment of the present invention, in-vivo biopsy, or fluid samples results collected in the satellite site 200 may be processed and/or analyzed in the central site with the central system 100. Other suitable methods of processing information may be performed by the central site system 100.

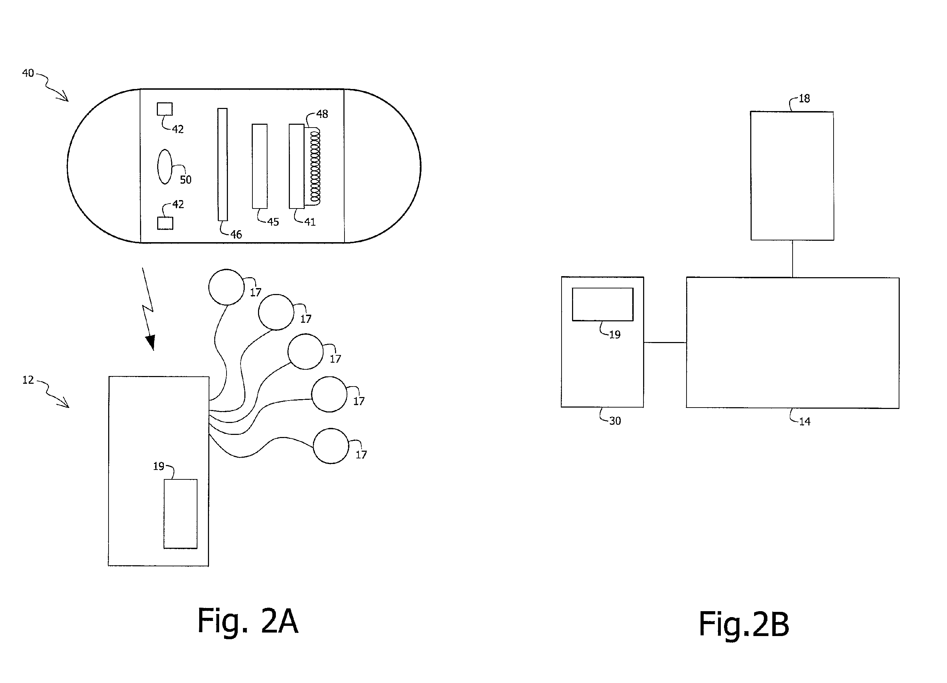

Reference is now made to Fig. 2A showing a schematic illustration of a system provided in a satellite site to capture and record in-vivo image data obtained from a swallowable imaging device passing through the GI tract according to an embodiment of the present invention. In some embodiments of the present invention a swallowable in-vivo device 40 may include, for example, an imager 46, an optical system 50, one or more illumination sources 42, and a power source 45. In some embodiments of the present invention data captured by the imager 46 may be transmitted by a transmitter 41 and an antenna 48, for example, by wireless communication, e.g. radio communication. Data transmitted from device 40 may be picked up by one or more antennas 17, for example an antenna array that may, for example, at least partially surround the patient. Other suitable methods of picking up data transmitted by device 40 may be used. The one or more antennas 17 may be connected by, for example, electrical communication to a recording device 12 which may include the storage unit 19. The recording device 12 may be a portable device that may be worn by the patient during data acquisition. In other embodiments of the present invention, device 12 may be part of a workstation or other suitable device. In some embodiments of the present invention, the storage unit 19 may be removable. For example, the storage unit 19 may be a portable hard disk, for example, PC card, disk on key, flash memory card or other suitable type of removable units that may include data storage capabilities. In some embodiments of the present invention, upon completion of data acquisition, the recording device 12 or the storage unit 19 or information stored on storage unit 19 may be sent by mail or may be hand delivered to a central site for downloading and processing. In other embodiments of the present invention, data recorded and stored in storage unit may be sent via internet and/or intranet communication. Other suitable means of data transfer may be used.

It is noted that some embodiments of the present invention may be directed to an autonomous, typically swallowable in-vivo device. Other embodiments need not be swallowable. Devices or systems according to embodiments of the present invention may be similar to embodiments described in US Publication No. 20010035902 published on November 1, 2001 and/or in U.S. Patent No. 5,604,531 published on February 18, 1997, each of which are assigned to the common assignee of the present invention and each of which are hereby fully incorporated by reference. Furthermore, a receiving and/or display system suitable for use with embodiments of the present invention may also be similar to embodiments described in US Publication No. 20010035902 and/or in U.S. Patent Number 5,604,531. Devices and systems as described herein may have other configurations and other sets of components. Alternate embodiments of a device, system and method according to various embodiments of the invention may be used with other devices, non-imaging and/or non-in-vivo devices.

According to some embodiments the recording device 12 and/or the storage unit 19 may be initialized at the satellite site 200. The initialization process may include entering patient data or other identification means that should typically be appended to the in vivo data being collected. Device 12 and/or storage unit 19 may include inputting means for such identification means to be entered by, for example, the patient or physician at the satellite site. Alternatively or in addition, a satellite site 200 may be provided with appropriate software that may be used by a PC or other means to enable initialization of the recording device 12 and/or the storage unit 19 prior to the data capturing process.

In some embodiments of the present invention, the initialization process may include one or more of the following steps:
(i) Checking if the recording device 12 has already undergone previous initialization. The user may be prompted for approval to continue the initialization. The user may exit the initialization process at this stage without deleting data.
(ii) Checking if the recording device 12 has already downloaded data. The user may be prompted for approval to continue the initialization. The user may exit the initialization process at this stage without deleting data.
(iii) Checking for new software and hardware upgrades and performing the upgrades upon confirmation by the user. On completion of upgrade, the recording device may be removed from the cradle and automatically shuts itself down.
(iv) Erasing the storage of the recording device 12.
(v) Entering of patient information, which may include, for example, patient data such as the name of the patient, gender of the patient, the birth date of the patient. Site data, including details of the geographical location of the site, may also be entered.
(vi) Performing of built in tests and checking storage medium for bad sectors. If the built in tests fail, or if there are bad sectors, then the initialization process will be terminated.
(vii) Checking the battery cycle count & manufacture date.
(viii) Updating the real time clock in the recording device 12.

Step (i) of the initialization process may be included to ensure that if a given recording device has already undergone initialization and therefore it is in a state ready for receiving in-vivo data from a given patient, that it will not be mistakenly re-initialized by another patient thereby giving rise to the possibility that the in-vivo data to be received at a later time will include the wrong patient identity. Step (ii) of the initialization process may be included to ensure that if a given recording device has already undergone both initialization and storage of in-vivo data, that it will not be accidentally erased. Step (iii) may be used not only for checking and performing new software and hardware upgrades, but also in cases in which problems are encountered due to bugs which may have formed in the software or hardware.

Following the data capturing process recording device 12 and/or the storage unit 19 which include identification data (e.g., patient data, site data, etc.) and in-vivo data may be sent to the central site 100. According to some embodiments the information, including identification data and in vivo data may be sent in electronic format to the central site 100.

Reference is now made to Fig. 2B showing a schematic illustration of a system provided in a central site for downloading and processing data captured in-vivo according to an embodiment of the present invention. In one embodiment of the present invention, a display unit or monitor 18 and a processing unit 14 may be, for example, a workstation with dedicated software and/or it may be another suitable processing unit. An interface unit 30 may be used to download data from the storage unit 19 to the processing unit 14. Unit 30 may be a cradle where storage unit 19 or recorder 12 may be positioned and may provide the interface protocol for communication between processing unit 14 and storage unit 19. Other suitable interfaces may be used to download information from storage unit 19 and or recording device 12. Subsequent to downloading, data downloaded may be further processed. In one embodiment of the present invention, a health professional may review the data downloaded and prepare a report based on findings. In one example, the health professional may mark specific image frames that may be relevant to a diagnosis. In another example, the health professional may review the downloaded data and give indication if a specified condition may have been visible and or discernable in the data, for example, the presence of blood, polyps, or other pathological indications. In one embodiment of the present invention, one or more dedicated algorithms may be implemented to decipher pathological conditions or other conditions of interest from the downloaded data. Typically, in some embodiments of the present invention, downloaded data with fmdings may be sent back to the satellite site for review and possibly further analysis by the health care professional or by the patient's physician. In one example, data may be sent by disk, via internet, intranet or other suitable means. In some embodiments of the present invention, a system similar to that shown in Fig, 2B is also provided at the satellite site 200. In some embodiments, the system similar to that shown in Fig, 2B which is also provided at the satellite site 200 may not be capable of processing data captured in-vivo, but may only be capable of carrying out the initialization process on the recording device 12 during which the recording device 12 is located in unit 30 (for example, in a cradle.

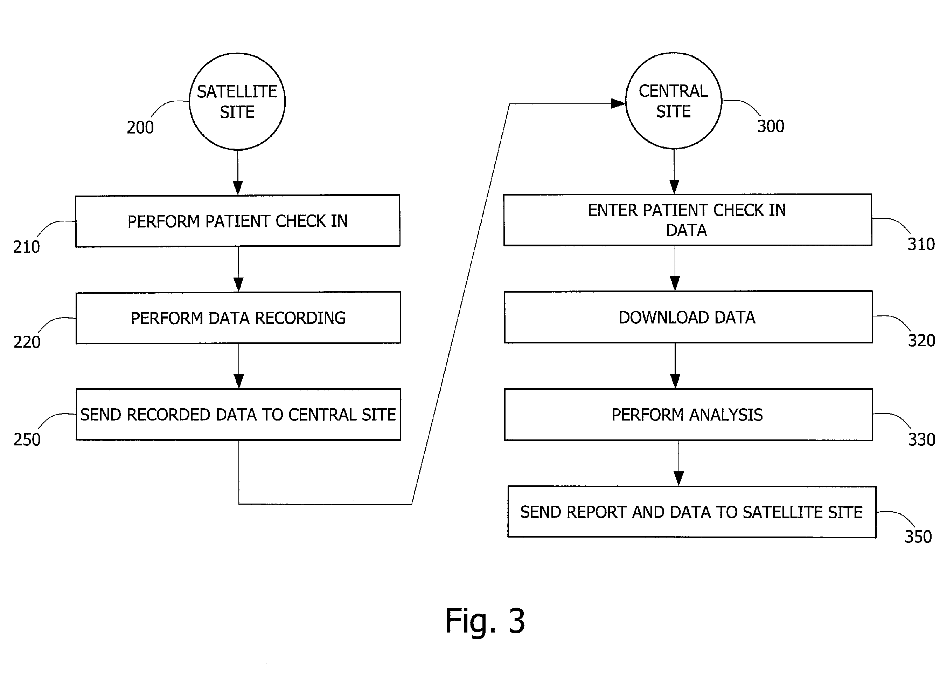

Reference is now made to Fig. 3 showing a flow chart describing a method for recording in-vivo data in a satellite site and downloading and processing the in-vivo data in a central site according to an embodiment of the present invention. A patient may come to a satellite site (200) to perform a capsule endoscopy procedure. Typically, patient check-in (210) involves initializing the recording device 12 that may include recording the patient's name, ID, medical history, medical condition and any other relevant data may be performed in the satellite site. In block 220, typically, a device 40 may be swallowed and data acquisition of in-vivo data from the capsule may begin. Data acquisition and recording may be performed, for example as may be described in US Publication No. 20010035902 and/or in U.S. Patent No. 5,604,531 or in other suitable manners. Acquired or recorded data may be sent (block 250), for example, with patient check-in info, to a central site 300 for downloading, processing and according to some embodiments, analyzing. In some embodiments of the present invention, central site 300 may be for example, a hospital, or a reading center with health professionals that may be trained to review data acquired from an in-vivo sensing device 40, or other suitable site. A central site may have dedicated equipment for downloading and/or processing acquired data. In some embodiments, if patient check-in data was not previously recorded in, for example, storage unit 19, the central site may enter patient data received from the satellite site (block 310). Data acquired may be downloaded (320) and analysis on the data may be performed. In block 350 downloaded data, together with patient check-in data and other analysis performed may be included in a report that may be sent to the satellite site. In addition, the recording device 12 and or storage unit 19 may be reformatted and sent to the satellite site for subsequent use, for example, on a new patient.

In one embodiment of the present invention, patient check-in data may be attached, for example in a hard copy format to storage unit 19 at the termination of the data acquisition procedure and prior to sending data acquired to the central site. In other embodiments of the present invention, satellite site 200 may be equipped with a unit to enable saving of patient check-in data directly into storage unit 19. For example satellite site 200 may be equipped with an interface unit 30 that may enable transfer of data from for example a personal computer to the recording unit 12 or directly to the storage unit 19. According to some embodiments satellite site 200 may use special software to enable initialization of the recording unit 12 and/or the storage unit 19, by which patient information may be entered. Other suitable means of recording patient check-in data and/or matching patient check-in data to the in-vivo data acquired from the patient may be used. In other embodiments of the present invention, patient check-in data may be recorded via internet and/or intranet that may be accessible to the central site. For example, patient check-in data together with data to identify the recording device 12 and storage unit 19 and/or the procedure code may be used to match the patient with the captured or acquired data. Other suitable methods may be used.

According to an embodiment of the invention raw data obtained from a swallowable capsule at a receiver/recorder in a satellite site may be initially processed and then transferred to the central site. At the central site the data (either raw data or initially processed data) is then subsequently processed. Typically, initial processing is less complicated processing than subsequent processing. Typically, a satellite site uses less computational power for processing raw data (initial processing) than the computational power utilized for processing data at the central site subsequent processing).

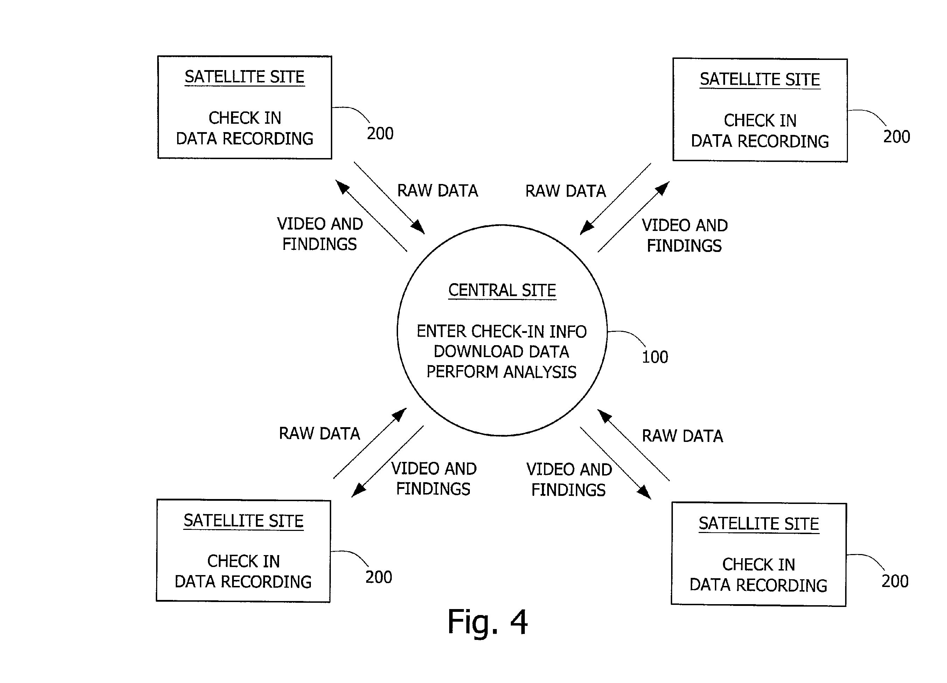

Reference is now made to Fig. 4 showing a block diagram of a method and system of diagnosis of a GI condition where in-vivo image capture may be performed in a plurality of satellite sites and downloading and procession of the captured data may be performed in a central site. One or more satellite sites 200 may send to a single central site 100 raw data or initially processed data. Central site 100 may in turn download the data, subsequently process data and send back to the respective satellite sites the subsequently processed image data, for example, in video format, or other format as well as findings or an analysis report. For example, initial processing may include image compression, editing and other relatively simple, low computing image processing algorithms. Subsequent processing may include image merging, color and/or pattern recognition and other high computing algorithms. Typically, for example, in one or more satellite sites initialization of a data recorder may be performed, which may include, erasing former data from the recorder and/or storage of the recorder and/or initial patient check-in. In the central site patient check-in may be completed, for example, by entering patient check-in information into the data acquired, downloading of data may be performed, and analysis either partial or complete may be performed. In some embodiments of the present invention, satellite sites may perform capsule endoscope diagnosis with minimal equipment and without training in reviewing data acquired by an in-vivo device. In addition the health care professional may not need to devote the time required for downloading and well as reviewing the image stream acquired.

It will be appreciated by persons skilled in the art that the present invention is not limited to what has been particularly shown and described hereinabove. Alternate embodiments are contemplated which fall within the scope of the invention.

## Claims

1. A method for performing capsule endoscopy on a patient located at a satellite site remote from a central site, comprising the steps of:
initializing a recording device located external to the patient;
obtaining in-vivo data of the patient from a capsule swallowed by the patient, the in-vivo data being transmitted wirelessly from the capsule to the recording device;
providing the in-vivo data from the recording device to the central site; and
processing the in-vivo data at the central site.

2. The method according to claim 1, wherein the step of providing the in-vivo data to the central site comprises transporting the recording device or a removable and/or mobile memory device which contains information from the recording device from the satellite site to the central site.

3. The method according to claim 1, wherein the step of providing the in-vivo data to the central site comprises transmitting the in-vivo data from the recording device at the satellite site to the central site via the Internet.

4. The method according to claim 1, wherein the step of providing the in-vivo data to the central site comprises transmitting the in-vivo data from the recording device at the satellite site wirelessly to the central site.

5. The method according to claim 1 comprising the step of transferring the in vivo data from the recording device to a portable memory device.

6. The method according to claim 1, wherein the step of initializing a recording device comprises entering patient information data on the recording device.

7. A system for performing capsule endoscopy comprising:
a satellite site at which a patient located, the satellite site being provided with software for performing initialization of recording devices;
a recording device that has undergone an initialization process at the satellite site and
which is located external to the patient, for receiving wirelessly and for storing in-vivo data generated by a capsule swallowed by the patient; and
a central site remote from the satellite site for analyzing in-vivo data stored in the recording device, only the central site being provided with software for processing the in-vivo data received from the satellite site.

8. The system according to claim 7, wherein the recording device is located at the central site after the storing of in-vivo data generated by the capsule swallowed by the patient at the satellite site.

9. The system according to claim 7, further comprising a portable memory device for receiving in-vivo data from the recording device.

10. The system according to claim 7 wherein the in vivo data includes image data.

11. A system for performing capsule endoscopy, the system comprising:
a swallowable imaging capsule said capsule wirelessly transmitting image data;
a receiver/recorder to receive the image data;
an initializing device to initialize the receiver/recorder; and
a processor to process the received image data;
wherein the initializing device and the processor are at remote locations from each other.

12. The system according to claim 11 wherein the initializing device is configured to initially process received image data, thereby producing initially processed image data and wherein the processor is configured to subsequently process the initially processed image data.
